(19) Europäisches Patentamt
European Patent Office
Office européen des brevets

(11) Veröffentlichungsnummer: **0 236 390**
**B1**

(12) # EUROPÄISCHE PATENTSCHRIFT

(45) Veröffentlichungstag der Patentschrift: **30.01.91**

(51) Int. Cl.[5]: **A 61 K 31/685**

(21) Anmeldenummer: **86905280.3**

(22) Anmeldetag: **28.08.86**

(86) Internationale Anmeldenummer:
**PCT/EP86/00506**

(87) Internationale Veröffentlichungsnummer:
**WO 87/01257 12.03.87 Gazette 87/06**

(54) **MITTEL GEGEN MULTIPLE SKLEROSE.**

(30) Priorität: **28.08.85 DE 3530767**

(43) Veröffentlichungstag der Anmeldung:
**16.09.87 Patentblatt 87/38**

(45) Bekanntmachung des Hinweises auf die Patenterteilung:
**30.01.91 Patentblatt 91/05**

(84) Benannte Vertragsstaaten:
**AT BE CH DE FR GB IT LI LU NL SE**

(56) Entgegenhaltungen:
**DE-A-2 619 686**

**Neurology (1983), Seite 73-80**

**Die Akte enthält technische Angaben, die nach dem Eingang der Anmeldung eingereicht wurden und die nicht in dieser Patentschrift enthalten sind.**

(73) Patentinhaber: **Max-Planck-Gesellschaft zur Förderung der Wissenschaften e.V.**
**Bunsenstrasse 10**
**D-3400 Göttingen (DE)**

(72) Erfinder: **MUNDER, Paul, Gerhard**
**Bergstrasse 38 a**
**D-7803 Gundelfingen (DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel**
**Möhlstrasse 22 Postfach 860 820**
**D-8000 München 86 (DE)**

**EP 0 236 390 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft die Verwendung von Verbindungen des Lysolecithin-Typs zur Herstellung von Mitteln für die Behandlung der Multiplen Sklerose.

In der DE—A—20 09 342 und der DE—A—20 09 343 ist die Verwendung von synthetischen Lysolecithin-Verbindungen zur Steigerung der Resistenz und als immunologische Adjuvantien beschrieben. Aus der DE—A—26 19 686 ist auch die Wirksamkeit derartiger Verbindungen als Anti-Tumormittel bekannt. Außerdem kommt es nach Applikation von Lysophosphatiden zur Bildung von aktivierten Zellen, die die Resistenz des Körpers gegen schädliche Einflüsse zu steigern vermögen.

Es wurde nun überraschend gefunden, daß Lysolecithin-Analoga der allgemeinen Formel I auch eine gute Wirksamkeit bei der Behandlung der Multiplen Sklerose zeigen.

Gegenstand der Erfindung ist deshalb die Verwendung einer Verbindung der allgemeinen Formel I

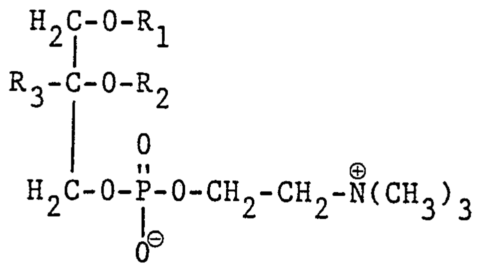

(I)

in der $R_1$ Alkyl mit 12 bis 18, insbesondere 16 bis 18 Kohlenstoffatomen, $R_2$ Alkyl mit 1 bis 8 Kohlenstoffatomen, $R_3$ H oder Alkyl mit 1 bis 3 Kohlenstoffatomen darstellt, zur Herstellung von Mitteln für die Behandlung der Multiplen Sklerose.

Eine Alkylgruppe $R_1$, $R_2$ und/oder $R_3$ kann verzweigt sein, und ist vorzugsweise geradkettig.

In einer Verbindung der allgemeinen Formel I ist $R_2$ vorzugsweise ein Alkylrest mit 1 bis 3 Kohlenstoffatomen, und insbesondere Methyl, und/oder $R_3$ vorzugsweise H. Bevorzugte Verbindungen sind insbesondere die Verbindung der Formel I mit $R_1$ = n-Hexadecyl, und $R_2$ = Methyl und $R_3$ = H (Verbindung II), und in erster Linie die Verbindung der Formel I mit $R_1$ = n-Octadecyl, und $R_2$ = Methyl und $R_3$ = H (Verbindung III).

Die Herstellung der Verbindungen der Formel I kann auf einem der in der Literatur beschriebenen Wege erfolgen; vgl. z.B. D. Arnold et al, Liebigs Ann. Chem. *709*, 234—239 (1967); H. U. Weltzien und O. Westphal, Liebigs Ann. Chem. *709*, 240—243 (1967); und H. Eibl und O. Westphal, Liebigs Ann. Chem. *709*, 244—247 (1967).

Multiple Sklerose (MS), eine der häufigsten Nevenkrankheiten, ist eine Autoimmunerkrankung, wobei T-Lymphozyten in die Nervensubstanz eindringen und dort das Myelin abbauen.

Im Tierversuch kann MS künstlich erzeugt werden, indem man aus Myelin das sog. basische Protein gewinnt und dieses zusammen mit komplettem Freund'schem Adjuvans Versuchstieren (Mäusen) injiziert. Nach 5 bis 7 Tagen werden aus den Tieren Lymphozyten gewonnen. Die aus dem Tier gewonnenen Lymphozyten können, wenn sie einem Versuchstier verabreicht werden, wobei mindestens $10^6$-T-Zellen pro Tier verabeicht werden müssen, MS auslösen (vgl. Ben-Nun A., Wekerle and Cohen I. R. The rapid isolation of clonable antigen specific T-lymphocyte capable of mediating autoimmune encephalomyletitis Eur. J. Immunology (1981), *11* 195).

Wird nun den Tieren eine Verbindung der allgemeinen Formel I, z.B. die Verbindung III, verabreicht, so lassen sich die MS-Symptome beseitigen. Daraus ist zu schließen, daß die Verbindungen der Formel I offenbar diejenigen T-Zellen-Klone in irgendeiner Weise inaktivieren, welche die die MS-Symptome hervorrufende Spezifität gegen Myelin oder basisches Protein entwickelt haben.

Auch am an MS erkrankten Menschen zeigte sich nach Verabreichung der Verbindung III eine deutliche Besserung der MS-Symptome: Bei oraler Verabreichung (Lösung der Verbindung III in lipoproteinhaltigen Flüssigkeiten, z.B. in Milch) konnte nach einer täglichen Verabeichung von 200 mg während mehrerer Wochen und danach Übergang auf eine nur jeden zweiten Tag erfolgende Verabreichung von 200 mg völlige Beschwerdefreiheit erreicht und beibehalten werden, was sich z.B. in der Fähigkeit zu einer völlig normalen Bewegung, zum Autofahren usw. äußerte.

Die Anwendung der erfindungsgemäß erhältlichen Mittel kann in der Regel auf eine übliche Art und Weise erfolgen, z.B. in der Art und Weise der Verabreichung derartiger Mittel als Anti-Tumormittel, also z.B. intravenös, peroral oder als Infusion; die orale Verabreichung ist aus Zweckmäßigkeitsgründen und bei einer über längere Zeit erforderlichen Applikation in der Regel die bevorzugte Anwendungsart.

Die Verabreichungsform (pharmazeutische Zubereitung) kann übliche Zusatzstoffe, wie übliche pharmazeutische Konfektionierungs- und/oder Verdünnungsmittel enthalten, und ggf. noch weitere Wirkstoffe, sofern diese mit den übrigen Bestandteilen keine unerwünschten Nebenwirkungen zeigen und zur Unterstützung der Therapie geeignet sind.

In der Regel liegt die wirksame Dosis für den Wirkstoff der erfindungsgemäß erhältlichen Mittel zwischen 1 und 10 mg/kg Körpergewicht/Tag, was bei der Verabreichung beim Menschen einer Dosis von ca. 100 mg bis 500 mg/Tag entspricht; vorzugsweise liegt die Dosis bei 150 bis 300 mg/Tag, wobei sich die Menge und Häufigkeit der Verabreichung aber auch nach dem Allgemeinbefinden des Patienten und der Schwere der Erkrankung richtet.

Beispiel 1

Eine Patientin, bei der vor 4 Jahren Multiple Sklerose diagnostiziert worden war und die unter einer spastischen Parese des rechten Beines und Schwindelgefühl litt, wurde jahrelang mit Kortison und Imurek® behandelt. Zusätzlich wurden krankengymnastische Übungen durchgeführt. Dadurch kam es zu einer langsamen Besserung, jedoch konnte nur eine kurze Wegstrecke von ca. 20 Minuten zurückgelegt werden. Außerdem bestand im Allgemeinbefinden eine starke Witterungsabhängigkeit. Nach 2 Jahren trat ein neuerlicher Schub auf. Dabei kam es zu Sensibilitätsstörungen beider Arme und des rechten Beines, zu Schreibschwierigkeiten, starker Gangunsicherheit im rechten Bein, starker Verkürzung der Wegstrecke, so daß die Wohnung praktisch nicht mehr verlassen werden konnte. Außerdem traten vorübergehend Sehstörungen und Schwindelgefühl auf. Trotz 5-monatiger Einnahme hoher Kortisondosen trat keine Besserung ein. In diesem Stadium wurde mit der Verabreichung der Verbindung III, die als ET—18—$OCH_3$ bezeichnet wird, begonnen. Es wurden täglich 200 mg dieser Verbindung, aufgelöst in Milch, verabreicht. Nach 14 Tagen dieser Behandlung konnte die Kortisontherapie beendet werden. Die Sensibilitäts- und Sehstörungen verschwanden. Die Patientin erhielt weiterhin 200 mg des Medikamentes ET—18—$OCH_3$ pro Woche. Diese Therapie wurde für weitere $1\frac{1}{2}$ Jahre durchgeführt. Die Wegstrecke beträgt jetzt mit kleinen Pausen ca. 4 Stunden. Klimatische Veränderungen, wie Aufenthalt an der See oder im Gebirge beeinträchtigen den Allgemeinzustand nicht mehr. Erkennbare Nebenwirkungen sind nicht aufgetreten. Die Patientin ist wieder gesund und arbeitsfähig.

Beispiel 2

Es wurde die Wirkung von verschiedenen Dosen von ET—18—$OCH_3$ auf MS-Zellen untersucht. Es wurden autoaggressive Lymphozyten, die Multiple Sklerose auslösen, von Ratten gewonnen (Ben-Nun A., Wekerle and Cohen I. R. The rapid isolation of clonable antigen specific T-lymphocyte capable of mediating autoimmune encephalomyletitis Eur. J. Immunology (1981), *11* 195). Diese Zellen wurden in bekannter Weise kultiviert und mit ET—18—$OCH_3$ behandelt. Nach 1, 2 und 3 Tagen wurde die Zellzahl bestimmt. Parallel wurde gelabelt durch Einbau von $^3$H-Thymidin. Die Ergebnisse sind der Figur 1 zu entnehmen. Es zeigt sich, daß durch die Behandlung mit ET—18—$OCH_3$ MS-Zellen zerstört werden. Bei Dosen von 3 mg/ml bis 10 mg/ml sind nach 3 Tagen praktisch alle MS-Zellen zerstört.

**Patentansprüche**

1. Verwendung einer Verbindung der allgemeinen Formel I

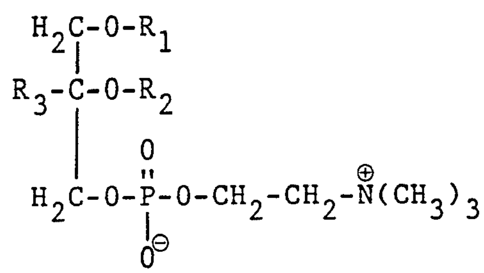

(I)

in der

$R_1$ Alkyl mit 12 bis 18 Kohlenstoffatomen,

$R_2$ Alkyl mit 1 bis 8 Kohlenstoffatomen und

$R_3$ H oder Alkyl mit 1 bis 3 Kohlenstoffatomen darstellt, zur Herstellung von Mitteln für die Behandlung von Multipler Sklerose.

2. Verwendung nach Anspruch 1, dadurch gekennzeichnet, daß in der allgemeinen Formel I $R_2$ eine Alkylgruppe mit 1 bis 3 Kohlenstoffatomen bedeutet

3. Verwendung einer Verbindung der allgemeinen Formel I mit der Struktur

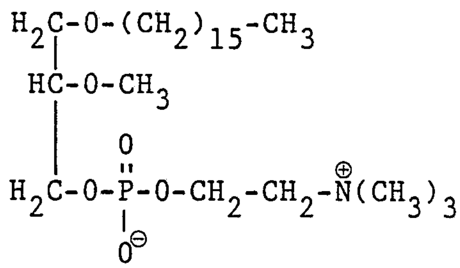

(II)

für den Zweck nach Anspruch 1.

4. Verwendung einer Verbindung der allgemeinen Formel I mit der Struktur

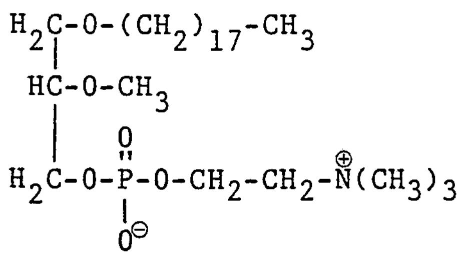

(III)

für den Zweck nach Anspruch 1.

**Revendications**

1. Utilisation d'un composé de formule générale I

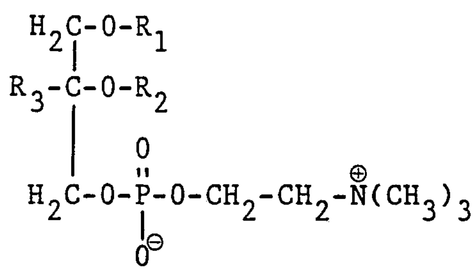

(I)

dans laquelle

$R_1$ représente un groupe alkyle de 12 à 18 atomes de carbone,

$R_2$ représente un groupe alkyle de 1 à 8 atomes de carbone, et

$R_3$ représente H ou un groupe alkyle de 1 à 3 atomes de carbone, pour la préparation d'agents pour le traitement de la sclérose multiple.

2. Utilisation selon la revendication 1, caractérisée en ce que, dans la formule générale I, $R_2$ représente un groupe alkyle de 1 à 3 atomes de carbone.

3. Utilisation d'un composé de formule générale I de structure

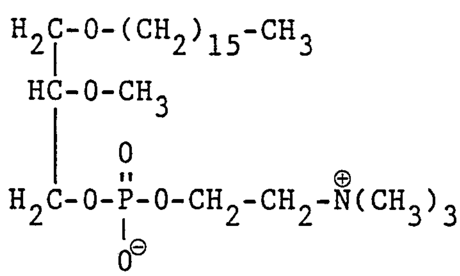

(II)

pour l'objet selon la revendication 1.

4. Utilisation d'un composé de formule générale I de structure

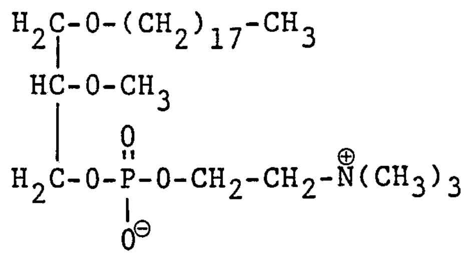

(III)

pour l'objet selon la revendication 1.

**Claims**

1. Use of a compound having the general formula I

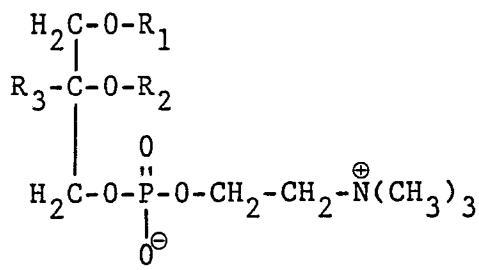

(I)

in which $R_1$ represents alkyl with 12 to 18 carbon atoms, $R_2$ alkyl with 1 to 8 carbon atoms and $R_3$ H or alkyl with 1 to 3 carbon atoms, for the preparation of agents for the treatment of multiple sclerosis.

2. Use according to claim 1, characterised in that, in the general formula I, $R_2$ signifies an alkyl group with 1 to 3 carbon atoms.

3. Use of a compound of the general formula I with the structure:

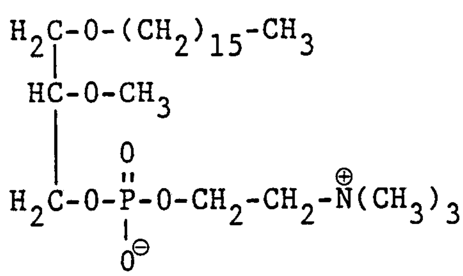

(II)

for the purpose according to claim 1.

4. Use of a compound of the general formula I with the structure:

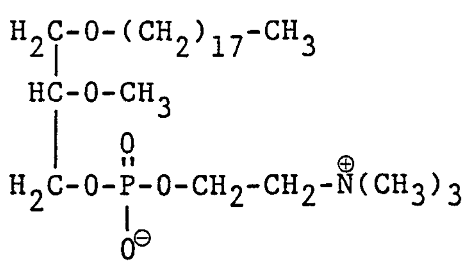

(III)

for the purpose according to claim 1.

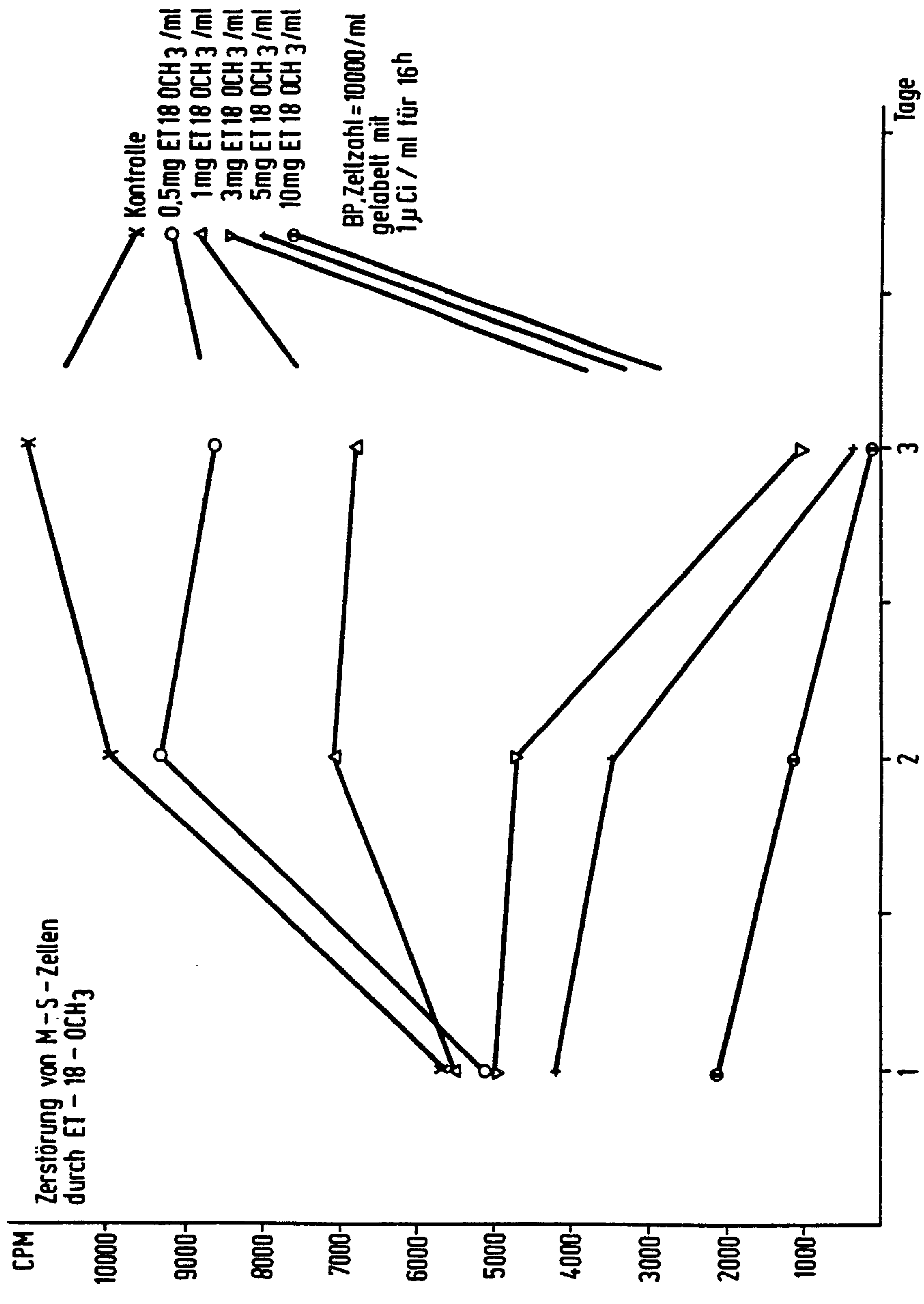
Zerstörung von M-S-Zellen
durch ET-18-OCH3
CPM
10000
9000
8000
7000
6000
5000
4000
3000
2000
1000
1
2
3
Tage
Kontrolle
0,5mg ET18 OCH 3 /ml
1mg ET18 OCH 3 /ml
3mg ET18 OCH 3 /ml
5mg ET18 OCH 3/ml
10mg ET18 OCH 3/ml
BP,Zellzahl = 10000/ml
gelabelt mit
1 µCi / ml für 16h